# EUROPEAN PATENT APPLICATION

(11) **EP 4 813 419 A1**
(43) Date of publication of application: **30.09.2026**
(21) Application number: 24893215.4
(22) Date of filing: 04.11.2024
(51) Int. Cl.: A61M 5/20

(54) **EXCITATION ASSEMBLY FOR PRE-FILLED AUTOMATIC INJECTION PEN AND PRE-FILLED AUTOMATIC INJECTION PEN**

(30) Priority: 22.11.2023 CN 202311569958; 22.11.2023 CN 202311568334
(71) Applicant: Shanghai Innopac Medical Technology Co., Led., Shanghai 201605 (CN)
(72) Inventor: WANG, Yijun, Shanghai 201605 (CN); GUO, Rong, Shanghai 201605 (CN)
(74) Representative: karo IP
(86) International application number: PCT/CN2024/129578
(87) International publication number: WO 2025/108057

(57) **Abstract**

The present invention provides an activation assembly for a pre-filled auto-injector pen and a pre-filled auto-injector pen. The activation assembly for a pre-filled auto-injector pen comprises: a support frame fixed within a housing and configured to initially define initial positions of a push rod, a spring clip, and a protective sleeve; the push rod, mounted within the support frame and initially positioned by the support frame, and configured, after the protective sleeve is triggered, to be driven under driving of an activation spring to drive a pre-filled syringe to move so as to complete automatic needle insertion; the protective sleeve, mounted outside the support frame, initially engaged outside the support frame and capable, after being moved under driving of an activation sleeve, of triggering the push rod, and further capable, after movement, of restricting rearward movement of the activation sleeve for needle protection; the activation spring, mounted inside the push rod and configured, after triggering, to provide an ejection force to the push rod, and, after the push rod disengages from the spring clip, to provide an ejection force to the spring clip; and the spring clip, mounted between the support frame and the push rod, and ejected under driving of the activation spring after completion of injection to strike a rear cover to produce a sound.

## Description

### Technical Field

The present invention belongs to the field of auto-injection technology, and specifically relates to an activation assembly for a pre-filled auto-injector pen and a pre-filled auto-injector pen.

### Background Art

A pre-filled auto-injector pen generally comprises, for a pre-filled syringe, an injection start mechanism, an injection driving mechanism, an injection prompt mechanism, and a needle protection mechanism after injection completion. Regarding the injection start mechanism, a start button is generally arranged on a side surface at or near an end of the injection pen, and the start button is generally pressed by a thumb or squeezed by a palm to actuate the injection start mechanism, thereby causing the injection driving mechanism to drive injection. Regarding the injection driving mechanism, an elastic element such as a spring is generally employed, or a mechanism such as an electric drive is employed, to achieve the injection action of the syringe. Regarding the injection prompt mechanism, generally when a plunger of the syringe reaches a bottom of the syringe, i.e., when the injection action is completed, a corresponding audible, visual, tactile or other related prompt is generated by a relevant mechanism to indicate to the user that the injection has ended. Furthermore, some products (such as BD Intevia) are additionally provided with a needle-withdrawal prompt; when the plunger reaches the bottom of the syringe, a delay mechanism is activated, and after the delay mechanism delays for a certain time, an audible, visual, tactile or other related prompt is generated to indicate to the user to withdraw the needle. Regarding the needle protection mechanism after injection completion, generally a needle shield is provided on one side of the needle, so as to be activated and fixed after injection completion, thereby achieving shielding protection of the needle to prevent the auto-injector pen after use from injuring the user.

The existing auto-injector pens have the following drawbacks:
1. The needle protection mechanism after injection completion cannot be locked.
2. The existing injection prompt mechanism has the problem of false prompting.
3. The existing injection driving mechanism has the problem of being falsely triggered.

### Summary of the Invention

Aimed at the problems existing in the above background art, an object of the present invention is to provide an activation assembly for a pre-filled auto-injector pen and a pre-filled auto-injector pen which has reliable activation, has a reliable injection-completion reminder function, and can achieve needle protection.

The technical solution adopted by the present invention is as follows:
An activation assembly for a pre-filled auto-injector pen, comprising:
a support frame fixed within a housing and configured to initially define initial positions of a push rod, a spring clip, and a protective sleeve;
a push rod mounted within the support frame and initially positioned by the support frame, and configured, after the protective sleeve is triggered, to be driven under driving of an activation spring to drive a pre-filled syringe to move so as to complete automatic needle insertion;
a protective sleeve mounted outside the support frame, initially engaged outside the support frame and capable, after being moved under driving of an activation sleeve, of triggering the push rod, and further capable, after movement, of restricting rearward movement of the activation sleeve for needle protection;
an activation spring mounted inside the push rod via a spring clip and configured, after triggering, to provide an ejection force to the push rod, and, after the push rod disengages from the spring clip, to provide an ejection force to the spring clip;
a spring clip mounted between the support frame and the push rod and configured, after completion of injection, to be ejected under driving of the activation spring to strike a rear cover to produce a sound; and
a rear cover fixed to a rear end of the support frame and configured to be struck by the spring clip to produce a sound.

Further, the support frame is provided with a first positioning groove for cooperatively positioning with the protective sleeve initially; the support frame is provided, at a rear side of the first positioning groove, with a limiting portion for cooperatively positioning with the protective sleeve after movement; a front end of the support frame is provided with a positioning portion for cooperatively positioning with the spring clip initially; the support frame is provided with a first cantilever; an inner wall of the first cantilever is provided with a first positioning catch for cooperatively positioning with the push rod initially; the first cantilever is initially press-fitted by the protective sleeve such that the first positioning catch engages with the push rod; a rear end of the support frame is provided with a second positioning groove for fixing the rear cover; and an outer surface of the support frame is symmetrically provided with arc-shaped protrusions for spreading open the protective sleeve to abut against the activation sleeve.

Further, the protective sleeve has a deformable front end; the deformable front end is capable of being spread open under action of the support frame to abut against a rear end of the activation sleeve; a linkage portion is protrudingly provided on an outer side surface of the deformable front end; the linkage portion is driven by the rear end of the activation sleeve to drive the protective sleeve to move rearward; a second positioning catch is provided on an inner side of a rear end of the protective sleeve; the second positioning catch is capable of engaging with the support frame both initially and after movement; and the protective sleeve is provided with a first sliding slot for initially receiving the protrusions and providing spreading space for the first cantilever after movement.

Further, the push rod is provided with a third positioning groove for cooperative initial positioning with the first positioning catch.

Further, the spring clip has a longitudinal cross-section of an E-shaped structure; a front end of the spring clip is provided with an engagement portion for cooperatively positioning with the support frame; the engagement portion is initially press-fitted by the push rod and engaged with the support frame; a positioning rod is provided at a middle of the spring clip; and the positioning rod is threaded through the push rod and is configured to position and mount the activation spring.

A pre-filled auto-injector pen, comprising:
a housing having a hollow structure;
a pen cap engaged with a head of the housing and capable, when pulled off, of driving an inner protective rubber sleeve of a pre-filled syringe to detach therewith;
an activation sleeve movably mounted within the housing and having a return spring for resetting axially provided between the activation sleeve and the housing, a head of the activation sleeve being exposed outside the housing, and the activation sleeve being capable of triggering an activation assembly when moving from an initial position to a limit position;
a pre-filled syringe clamped on a fixing seat when pre-mounted, wherein during movement of the activation sleeve from the initial position to the limit position, an injection end of the pre-filled syringe is always concealed within a needle shield, and when the injection end of the pre-filled syringe is triggered, the pre-filled syringe is instantaneously displaced to extend beyond the activation sleeve to achieve automatic needle insertion;
a fixing seat fixedly mounted between the activation sleeve and the pre-filled syringe, configured to initially define an initial position of the pre-filled syringe and to provide support during automatic needle insertion of the pre-filled syringe;
wherein the activation assembly for a pre-filled auto-injector pen as described above is mounted within the housing.

Further, the head of the push rod is a pushing end in contact with a rubber plunger of the pre-filled syringe, and the interior of the push rod is a hollow structure for accommodating the activation spring.

Further, the activation sleeve comprises an activation sleeve body for protecting the needle; a tail end of the activation sleeve body is provided with symmetrically arranged activation portions; each of the activation portions is provided with a slot hole for movement of a second cantilever of the fixing seat; and an inner side of each of the activation portions is provided, at one end of the slot hole, with a protection catch protruding radially for defining an initial position of the second cantilever of the fixing seat.

Further, the fixing seat is symmetrically provided with second cantilevers; the second cantilevers are arranged corresponding to the slot holes of the activation sleeve; an outer side of an end portion of each of the second cantilevers is provided with an outer catch for initially cooperating with the protection catch of the activation sleeve to define a position of the second cantilever; and an inner side of the end portion of each of the second cantilevers is provided with an inner catch for initially cooperating with a protruding tail end of the pre-filled syringe to support the pre-filled syringe; an inner wall of the fixing seat is provided with a stepped surface for limiting movement of the pre-filled syringe; and the inner wall of the fixing seat is axially provided, from the stepped surface to a tail end thereof, with vertical ribs for providing support during automatic needle insertion of the pre-filled syringe.

Further, the housing is provided with a viewing window for inspecting a state of medicinal liquid, whether a position of the pre-filled syringe is correct, and an infusion condition after completion of injection.

Further, the pen cap is provided with symmetrically arranged pen cap catches, and the pen cap catches are initially snap-connected with the inner protective rubber sleeve of the pre-filled syringe.

Further, the rear cover is symmetrically provided with connecting catches for engaging with the second positioning groove on the support frame.

Compared with the prior art, the significant advantages of the present invention include:
1. Activation of the push rod can be achieved merely through movement of the protective sleeve, and the activation is reliable and stable.
2. After completion of push-injection by the push rod, the push rod disengages from the spring clip, and the spring clip is capable, under action of the activation spring, of moving rearward to strike the rear cover to produce a sound, thereby achieving the injection-completion reminder function and ensuring reliability of the end prompt.
3. The protective sleeve is capable of restricting rearward movement of the activation sleeve after injection completion, thereby achieving needle protection, preventing accidental injury, and disabling secondary use.
4. With viewing window function, the injection condition can be observed after the end prompt sound, ensuring continuous integrity of the injection.

### Brief Description of the Drawings

FIG. 1 is a schematic exploded structural view of Embodiment 1 of the present invention;
FIG. 2 is a first schematic cross-sectional structural view of Embodiment 1 of the present invention in an initial state;
FIG. 3 is a second schematic cross-sectional structural view of Embodiment 1 of the present invention in the initial state;
FIG. 4 is a schematic cross-sectional structural view of Embodiment 1 of the present invention after activation;
FIG. 5 is a schematic structural view of the support frame of Embodiment 1 of the present invention;
FIG. 6 is a schematic structural view of the protective sleeve of Embodiment 1 of the present invention;
FIG. 7 is a schematic structural view of the spring clip of Embodiment 1 of the present invention;
FIG. 8 is a schematic structural view of the push rod of Embodiment 1 of the present invention.
FIG. 9 is a schematic exploded structural view of Embodiment 2 of the present invention;
FIG. 10 is a schematic external structural view of Embodiment 2 of the present invention;
FIG. 11 is a first schematic cross-sectional structural view of Embodiment 2 of the present invention in an initial state;
FIG. 12 is a second schematic cross-sectional structural view of Embodiment 2 of the present invention in the initial state;
FIG. 13 is a schematic cross-sectional structural view of Embodiment 2 of the present invention after activation;
FIG. 14 is a schematic cross-sectional structural view illustrating the limiting between the activation sleeve and the protective sleeve after needle withdrawal in Embodiment 2 of the present invention;
FIG. 15 is a schematic structural view of the pen cap of Embodiment 2 of the present invention;
FIG. 16 is a schematic structural view of the housing of Embodiment 2 of the present invention;
FIG. 17 is a schematic structural view of the activation sleeve of Embodiment 2 of the present invention;
FIG. 18 is a schematic structural view of the pre-filled syringe of Embodiment 2 of the present invention;
FIG. 19 is a schematic structural view of the fixing seat of Embodiment 2 of the present invention;
FIG. 20 is a schematic structural view of the rear cover of Embodiment 2 of the present invention.

### Reference signs:

1 - pen cap; 1a - pen cap catch; 2 - housing; 21 - viewing window; 3 - activation sleeve; 31 - activation sleeve body; 32 - activation portion; 33 - slot hole; 34 - protection catch; 4 - return spring; 5 - pre-filled syringe; 51 - inner protective rubber sleeve; 52 - protruding tail end; 6 - fixing seat; 61 - second cantilever; 62 - outer catch; 63 - inner catch; 64 - stepped surface; 65 - vertical rib; 7 - support frame; 71 - first positioning groove; 72 - limiting portion; 73 - positioning portion; 74 - first cantilever; 75 - first positioning catch; 76 - second positioning groove; 77 - protrusion; 8 - push rod; 81 - third positioning groove; 82 - pushing end; 9 - activation spring; 10 - protective sleeve; 101 - deformable front end; 102 - linkage portion; 103 - second positioning catch; 104 - first sliding slot; 11 - spring clip; 111 - engagement portion; 112 - positioning rod; 12 - rear cover; 121 - connecting catch.

### Detailed Description of Embodiments

The present invention will be further described below in conjunction with specific embodiments, but the present invention is not limited to these specific embodiments. Those skilled in the art should recognize that the present invention encompasses all alternative solutions, modifications, and equivalents that may be included within the scope of the claims.

In the description of the present invention, it should be understood that the terms "center", "longitudinal", "transverse", "length", "width", "thickness", "upper", "lower", "front", "rear", "left", "right", "vertical", "horizontal", "top", "bottom", "inner", "clockwise", "counterclockwise" and the like indicate orientations or positional relationships based on the orientations or positional relationships shown in the drawings, and are merely for facilitating description of the present invention and simplifying description, rather than indicating or implying that the referred device or element must have a specific orientation or be constructed and operated in a specific orientation, and therefore cannot be construed as limiting the present invention. Furthermore, the terms "first" and "second" are used for descriptive purposes only and cannot be construed as indicating or implying relative importance or implicitly indicating the number of the technical features indicated. Thus, features defined with "first" or "second" may explicitly or implicitly include one or more such features. In the description of the present invention, unless otherwise specified, "a plurality of" means two or more, unless otherwise expressly defined.

In the present invention, unless otherwise expressly stipulated and defined, the terms "mount", "couple", "connect", "fix" and the like should be understood in a broad sense. For example, they may refer to a fixed connection, a detachable connection, or an integral connection; may be a mechanical connection or an electrical connection; may be a direct connection, or may be an indirect connection through an intermediate medium, or may be a communication between interiors of two elements. For those of ordinary skill in the art, the specific meanings of the above terms in the present invention can be understood according to specific circumstances.

In the present invention, unless otherwise expressly stipulated and defined, a first feature being "above" or "below" a second feature may include the first and second features being in direct contact, or may include the first and second features being not in direct contact but in contact through another feature therebetween. Moreover, the first feature being "above", "over" and "on top of" the second feature includes the first feature being directly above and obliquely above the second feature, or merely indicates that the horizontal height of the first feature is higher than that of the second feature. The first feature being "below", "under" and "beneath" the second feature includes the first feature being directly below and obliquely below the second feature, or merely indicates that the horizontal height of the first feature is less than that of the second feature.

### Embodiment 1

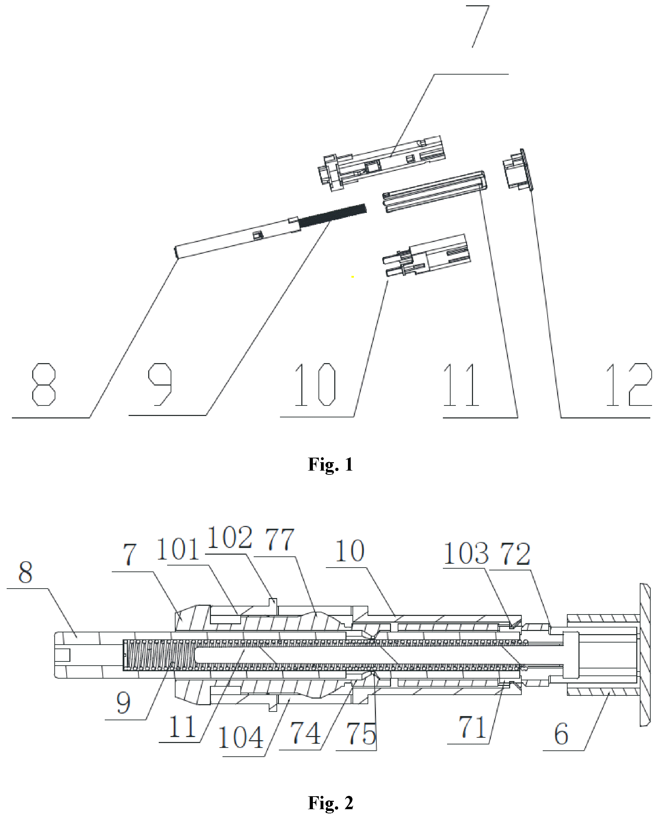

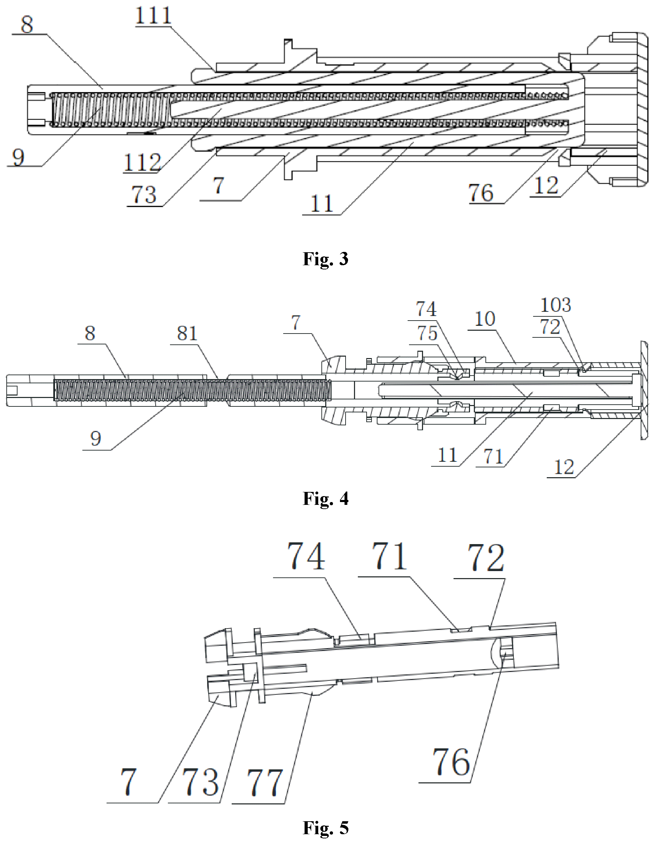

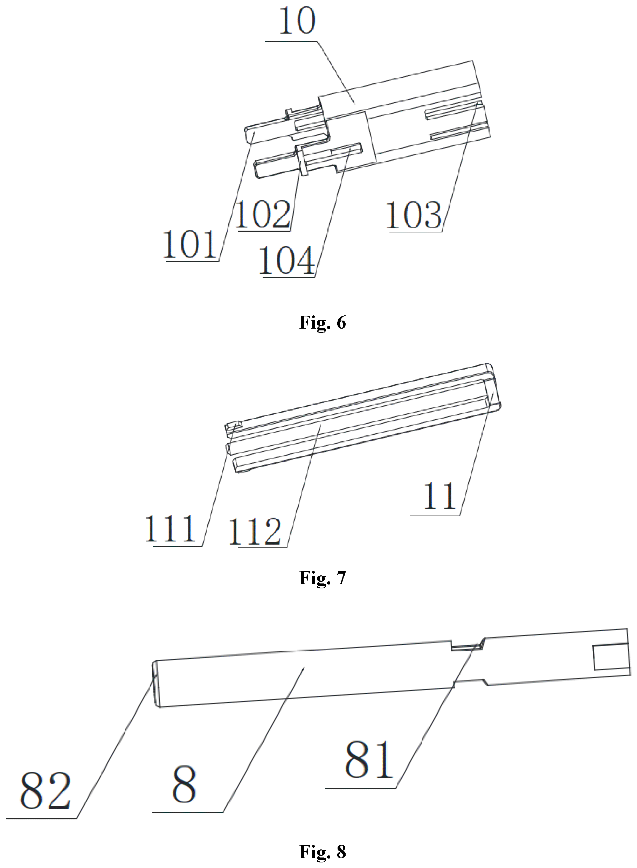

Referring to FIGS. 1-8, the present embodiment provides an activation assembly for a pre-filled auto-injector pen, comprising:
a support frame 7 fixed within a housing 2 and configured to initially define initial positions of a push rod 8, a spring clip 11, and a protective sleeve 10;
the push rod 8, mounted within the support frame 7 and initially positioned by the support frame 7, and configured, after the protective sleeve 10 is triggered, to be driven under driving of an activation spring 9 to drive a pre-filled syringe 5 to move so as to complete automatic needle insertion;
the protective sleeve 10, mounted outside the support frame 7, initially engaged outside the support frame 7 and capable, after being moved under driving of an activation sleeve 3, of triggering the push rod 8, and further capable, after movement, of restricting rearward movement of the activation sleeve 3 for needle protection;
the activation spring 9, mounted inside the push rod 8 via the spring clip 11 and configured, after triggering, to provide an ejection force to the push rod 8, and, after the push rod 8 disengages from the spring clip 11, to provide an ejection force to the spring clip 11;
the spring clip 11, mounted between the support frame 7 and the push rod 8 and configured, after completion of injection, to be ejected under driving of the activation spring 9 to strike a rear cover 12 to produce a sound; and
the rear cover 12, fixed to a rear end of the support frame 7 and configured to be struck by the spring clip 11 to produce a sound.

In the present embodiment, the support frame 7 is provided with a first positioning groove 71 for cooperatively positioning with the protective sleeve 10 initially; the support frame 7 is further provided, at a rear side of the first positioning groove 71, with a limiting portion 72 for cooperatively positioning with the protective sleeve after movement; a front end of the support frame 7 is provided with a positioning portion 73 for cooperatively positioning with the spring clip 11 initially; an inner wall surface of the support frame 7 is provided with a first positioning catch 75 for cooperatively positioning with the push rod initially; and a rear end of the support frame 7 is provided with a second positioning groove 76 for fixing the rear cover. The support frame 7 is provided with a first cantilever 74, and the first positioning catch 75 is provided on an inner side of the first cantilever 74; the first cantilever 74 is initially press-fitted by the protective sleeve 10 such that the first positioning catch 75 engages with the push rod 8. The push rod 8 is provided with a third positioning groove 81 for cooperative initial positioning with the first positioning catch 75. An outer surface of the support frame 7 is symmetrically provided with arc-shaped protrusions 77; the deformable front end 101 of the protective sleeve 10 is spread and deformed when moving to the protrusion 77.

A front end wall surface of the protective sleeve 10 in the present embodiment is provided with notches, thereby forming a deformable structure at the front end. The deformable front ends 101 are symmetrically arranged; the deformable front ends 101 are capable of being spread open under action of the support frame 7 to abut against a rear end of the activation sleeve; a linkage portion 102 is protrudingly provided on an outer side surface of the deformable front end 101; the linkage portion 102 is driven by the rear end of the activation sleeve to drive the protective sleeve 10 to move rearward; a second positioning catch 103 is provided on an inner side of a rear end of the protective sleeve 10; the second positioning catch 103 is capable of engaging with the support frame 7 both initially and after movement; and the second positioning catch 103 is arranged on a cantilever of the protective sleeve 10 to provide deformation space. The protective sleeve 10 is provided with a first sliding slot 104 for initially receiving the protrusion 77 and providing spreading space for the first cantilever 74 after movement. The arrangement of the first sliding slot 104 is such that the protective sleeve 10 does not deform during the initial state and during a front section of rearward movement, and is only spread and deformed when the deformable front end 101 moves to the protrusion 77 so as to be able to restrict rearward movement of the activation sleeve; moreover, when the first sliding slot 104 moves to the position of the first cantilever 74, the first cantilever 74 is able to be spread open by the push rod 8, such that the push rod 8 leaves the initial position and moves under action of the activation spring 9.

The spring clip 11 in the present embodiment has a longitudinal cross-section of an E-shaped structure; a front end of the spring clip 11 is provided with an engagement portion 111 for cooperatively positioning with the support frame 7; the engagement portion 111 is initially press-fitted by the push rod 8 and engaged with the support frame 7. A positioning rod 112 is provided at a middle of the spring clip 11; the positioning rod 112 is threaded through the push rod 8 and is configured to position and mount the activation spring 9.

In the present embodiment, an inner wall of the first positioning groove 71, an inner wall of the second positioning groove 76, an inner wall of the third positioning groove 81, and the engagement portion 111 are each provided with an inclined surface structure for disengagement from the initial position.

During assembly of the present invention, the protective sleeve 10 is mounted outside the support frame 7, at which time the second positioning catch 103 is engaged within the first positioning groove 71, the protrusion 77 is located within the first sliding slot 104, and the first cantilever 74 is press-fitted by the protective sleeve 10; the spring clip 11 is mounted into the support frame 7, at which time the engagement portion 111 is engaged with and positioned by the positioning portion 73; the activation spring 9 is sleeved on the positioning rod 112, and then the push rod 8 is mounted into the support frame 7, at which time the first positioning catch 75 is engaged within the third positioning groove 81; the rear cover 12 is engaged with the rear end of the support frame 7, and a connecting catch on the rear cover 12 is engaged within the second positioning groove 76.

During use of the present invention, when the activation sleeve moves rearward and contacts the linkage portion 102 of the protective sleeve 10, the protective sleeve 10 moves rearward under driving of the activation sleeve, and the second positioning catch 103 disengages from the first positioning groove 71; the protective sleeve 10 continues to move rearward, and when the second positioning catch 103 reaches the limiting portion 72, the first sliding slot 104 moves to the position of the first cantilever 74; the first cantilever 74 loses its press-fitting force; under action of the activation spring 9, the push rod 8 spreads the first positioning catch 75 outward to disengage from the third positioning groove 81, and the push rod 8 moves forward along the inner wall of the support frame 7; when the push rod 8 moves to a limit position, i.e., when injection is completed, the push rod 8 completely disengages from the spring clip 11; at this time, under action of the activation spring 9, the engagement portion 111 of the spring clip 11 disengages from the positioning portion of the support frame 7, and the spring clip 11 is ejected and strikes the rear cover 12 to produce a sound, indicating that injection has been completed.

Meanwhile, the deformable front end 101 of the protective sleeve 10 is spread and deformed when moving to the protrusion 77, thereby restricting the rearward movement path of the activation sleeve; that is, the activation sleeve, after needle withdrawal is completed, is in a state of protecting the needle, and cannot be operated for a second time or cause accidental injury.

In the present invention, activation of the push rod 8 can be achieved merely through movement of the protective sleeve 10, and the activation is reliable and stable. After completion of push-injection by the push rod 8, the push rod 8 disengages from the spring clip 11, and the spring clip 11 is capable, under action of the activation spring 9, of moving rearward to strike the rear cover 12 to produce a sound, thereby achieving the injection-completion reminder function and ensuring reliability of the end prompt. The protective sleeve 10 is capable of restricting rearward movement of the activation sleeve after injection completion, thereby achieving needle protection, preventing accidental injury, and disabling secondary use.

### Embodiment 2

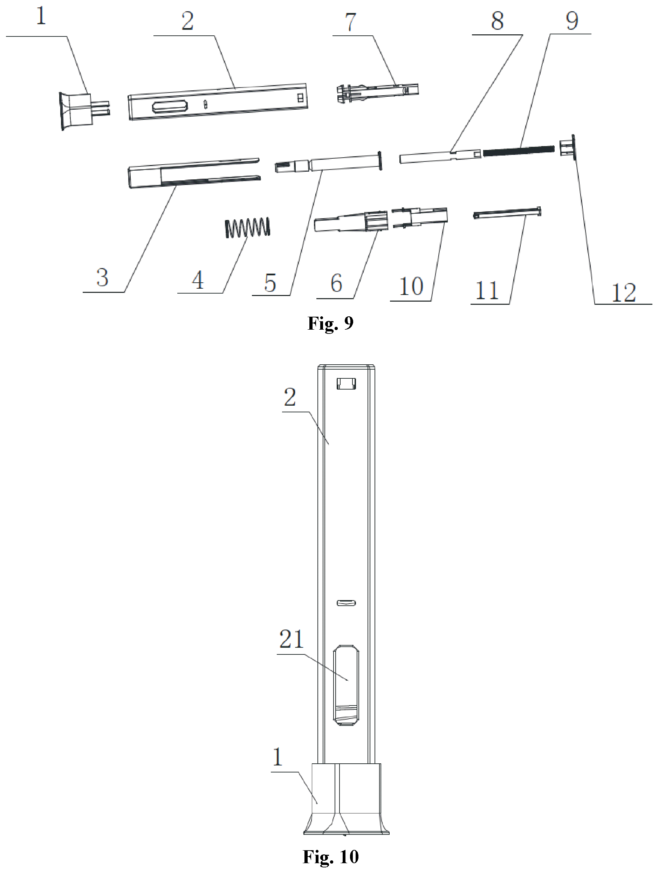

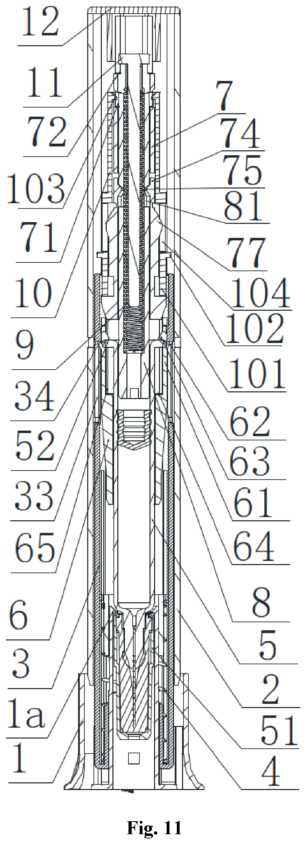

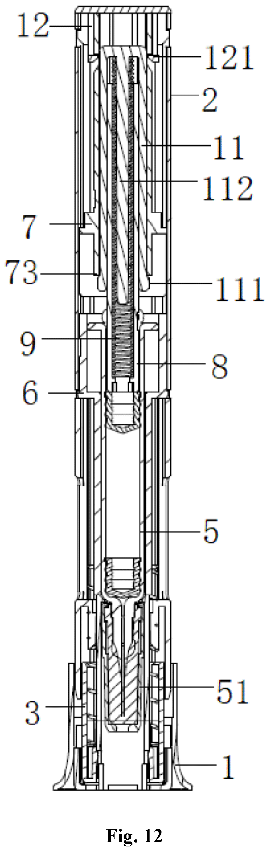

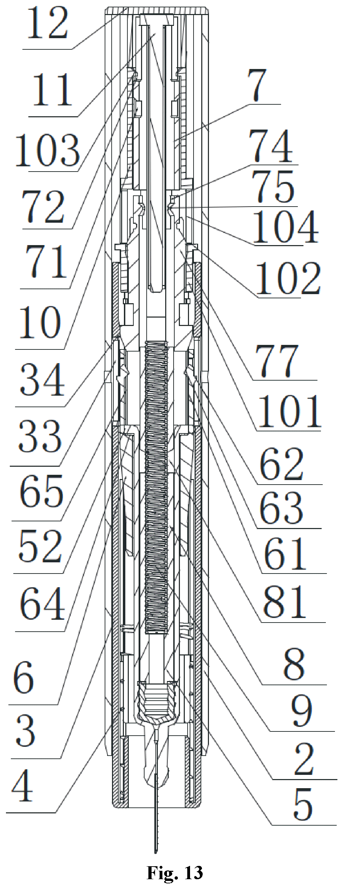

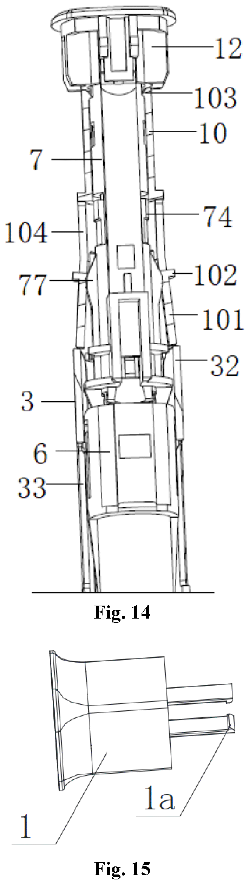

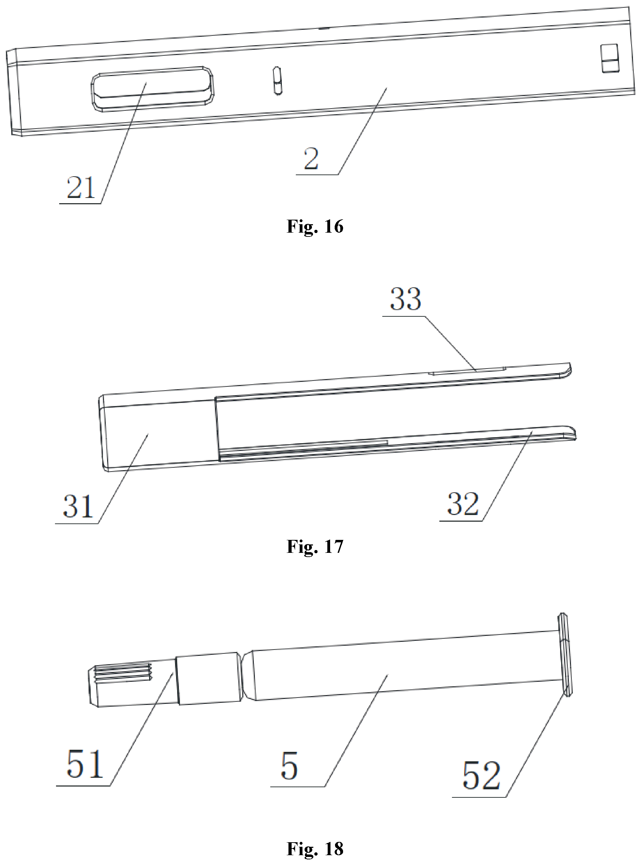

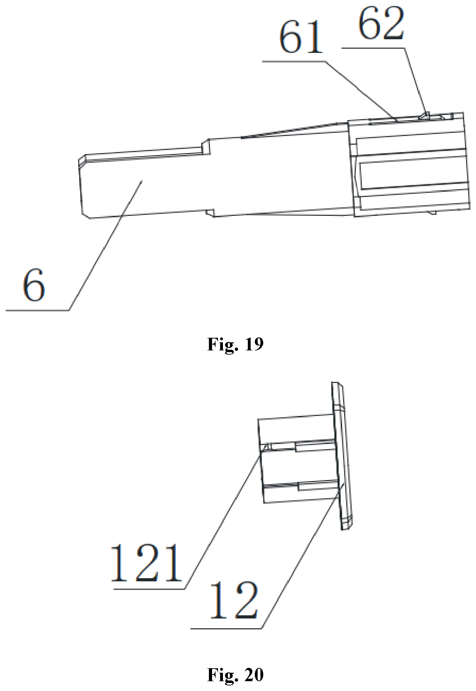

Referring to FIGS. 9-20, the present embodiment provides a pre-filled auto-injector pen, comprising a pen cap 1, a housing 2, an activation sleeve 3, a return spring 4, a pre-filled syringe 5, a fixing seat 6, a support frame 7, a push rod 8, an activation spring 9, a protective sleeve 10, a spring clip 11, and a rear cover 12, wherein the support frame 7, the push rod 8, the activation spring 9, the protective sleeve 10, the spring clip 11, and the rear cover 12 constitute an activation assembly, and the activation assembly is mounted within the housing 2.

In the present embodiment, the pen cap 1 is engaged with a head of the housing 2 and, when pulled off, is capable of driving an inner protective rubber sleeve of the pre-filled syringe 5 to detach therewith. Specifically, the pen cap 1 is provided with symmetrically arranged pen cap catches 1a; the pen cap catches 1a are initially snap-connected with the inner protective rubber sleeve 51 of the pre-filled syringe 5. The inner protective rubber sleeve 51 of the pre-filled syringe 5 disengages from the pre-filled syringe 5 when the pen cap 1 is pulled off, thereby exposing the needle.

In the present embodiment, the housing 2 has a hollow structure; the housing 2 is provided with a viewing window 21 for inspecting a state of medicinal liquid, whether a position of the pre-filled syringe is correct, and an infusion condition after completion of injection.

In the present embodiment, the activation sleeve 3 is movably mounted within the housing 2, with a return spring 4 for resetting being axially provided between the activation sleeve 3 and the housing 2; a head of the activation sleeve 3 is exposed outside the housing 2, and the activation sleeve 3 is capable, when moving from an initial position to a limit position, of triggering the activation assembly. Specifically, the activation sleeve 3 comprises an activation sleeve body 31 for protecting the needle; a tail end of the activation sleeve body 31 is provided, by extension, with symmetrically arranged activation portions 32; each of the activation portions 32 is provided with a slot hole 33 for movement of a second cantilever 61 of the fixing seat 6; and an inner side of each of the activation portions 32 is provided, at one end of the slot hole 33, with a protection catch 34 protruding radially for defining an initial position of the second cantilever 61 of the fixing seat 6. After a rear end of the activation portion 32 moves and contacts the linkage portion 102 of the protective sleeve 10, the protective sleeve 10 is driven to move rearward, thereby achieving activation of the activation assembly.

In the present embodiment, the pre-filled syringe 5 is clamped on the fixing seat 6 when pre-mounted; during movement of the activation sleeve 3 from the initial position to the limit position, an injection end of the pre-filled syringe 5 is always concealed within a needle shield; when the injection end of the pre-filled syringe 5 is triggered, the pre-filled syringe 5 is instantaneously displaced to extend beyond the activation sleeve 3 to achieve automatic needle insertion. Specifically, the position at which the pre-filled syringe 5 is clamped on the second cantilever 61 of the fixing seat 6 when pre-mounted is the initial position of the pre-filled syringe 5; during activation, the activation sleeve 3 moves from the initial position to the limit position; when the activation sleeve 3 is at the limit position, the injection end of the pre-filled syringe 5 is concealed within the activation sleeve 3; when the injection end of the pre-filled syringe 5 is triggered, the pre-filled syringe 5 is instantaneously displaced, moving from the initial position to a stepped surface 64 on the fixing seat 6 and projecting beyond the activation sleeve 3 to achieve automatic needle insertion; the injection end is a needle structure, initially provided with the inner protective rubber sleeve 51 for protecting the injection end; a liquid is pre-filled within the pre-filled syringe 5 and is sealed by a rubber plunger; a protruding tail end 52 of the pre-filled syringe 5 is protrudingly arranged, can be defined by the fixing seat 6 at the initial position, and cooperates with the stepped surface 64 on the fixing seat 6 to limit a movement distance.

In the present embodiment, the fixing seat 6 is fixedly mounted between the activation sleeve 3 and the pre-filled syringe 5, initially defines the initial position of the pre-filled syringe 5 and is capable of providing support during automatic needle insertion of the pre-filled syringe 5; the fixing seat 6 is symmetrically provided with second cantilevers 61; the second cantilevers 61 are arranged corresponding to the slot holes 33 of the activation sleeve 3; an outer side of an end portion of each of the second cantilevers 61 is provided with an outer catch 62 for initially cooperating with the protection catch 34 of the activation sleeve 3 to define a position of the second cantilever 61; an inner side of the end portion of each of the second cantilevers 61 is provided with an inner catch 63 for initially cooperating with the protruding tail end 52 of the pre-filled syringe 5 to support the pre-filled syringe 5; an inner wall of the fixing seat 6 is provided with the stepped surface 64 for limiting movement of the pre-filled syringe 5; and the inner wall of the fixing seat 6 is axially provided, from the stepped surface 64 to a tail end thereof, with vertical ribs 65 for providing support during automatic needle insertion of the pre-filled syringe 5. After the outer catch 62 of the second cantilever 61 of the fixing seat 6 disengages from the protection catch 34, the second cantilever 61 can deform within the slot hole 33, such that the pre-filled syringe 5 breaks through the inner catch 63 to move downward to the stepped surface 64 under action of the push rod 8. A silicone gasket may be arranged on the stepped surface 64 for protecting the protruding tail end 52 of the pre-filled syringe 5 to prevent breakage during movement.

In the present embodiment, the support frame 7 is fixed within the housing 2 and is configured to initially define the initial positions of the push rod 8, the spring clip 11, and the protective sleeve 10. Specifically, the support frame 7 is provided with a first positioning groove 71 for cooperatively positioning with the protective sleeve 10 initially; the support frame 7 is further provided, at a rear side of the first positioning groove 71, with a limiting portion 72 for cooperatively positioning with the protective sleeve 10 after movement; a front end of the support frame 7 is provided with a positioning portion 73 for cooperatively positioning with the spring clip 11 initially; the support frame 7 is provided with a first cantilever 74; an inner wall of the first cantilever 74 is provided with a first positioning catch 75 for cooperatively positioning with the push rod 8 initially; the first cantilever 74 is initially press-fitted by the protective sleeve 10 such that the first positioning catch 75 engages with the push rod 8; a rear end of the support frame 7 is provided with a second positioning groove 76 for fixing the rear cover 12; and an outer surface of the support frame 7 is symmetrically provided with arc-shaped protrusions 77 for spreading open the protective sleeve 10 to abut against the activation sleeve 3.

In the present embodiment, the push rod 8 is mounted within the support frame 7 and is initially positioned by the support frame 7, and is configured, after the protective sleeve 10 is triggered, to be driven under driving of the activation spring 9 to drive the pre-filled syringe 5 to move so as to complete automatic needle insertion. Specifically, the push rod 8 is provided with a third positioning groove 81 for cooperative initial positioning with the first positioning catch 75; a head of the push rod 8 is a pushing end 82 in contact with the rubber plunger of the pre-filled syringe 5; and an interior of the push rod 8 is a hollow structure for accommodating the activation spring 9.

In the present embodiment, the protective sleeve 10 is mounted outside the support frame 7, initially engaged outside the support frame 7 and capable, after being moved under driving of the activation sleeve 3, of triggering the push rod 8, and further capable, after movement, of restricting rearward movement of the activation sleeve 3 for needle protection. Specifically, a front end wall surface of the protective sleeve 10 is provided with notches, thereby forming a deformable structure at the front end. The deformable front ends 101 are symmetrically arranged; the deformable front ends 101 are capable of being spread open under action of the support frame 7 to abut against the rear end of the activation sleeve 3; the linkage portion 102 is protrudingly provided on an outer side surface of the deformable front end 101; the linkage portion 102 is driven by the rear end of the activation sleeve 3 to drive the protective sleeve 10 to move rearward; a second positioning catch 103 is provided on an inner side of a rear end of the protective sleeve 10; the second positioning catch 103 is capable of engaging with the support frame 7 both initially and after movement; and the protective sleeve 10 is provided with a first sliding slot 104 for initially receiving the protrusion 77 and providing spreading space for the first cantilever 74 after movement. The arrangement of the first sliding slot 104 is such that the protective sleeve 10 does not deform during the initial state and during a front section of rearward movement, and is only spread and deformed when the deformable front end 101 moves to the protrusion 77 so as to be able to restrict rearward movement of the activation sleeve 3; moreover, when the first sliding slot 104 moves to the position of the first cantilever 74, the first cantilever 74 is able to be spread open by the push rod 8, such that the push rod 8 leaves the initial position and moves under action of the activation spring 9.

In the present embodiment, the activation spring 9 is mounted inside the push rod 8 via the spring clip 11 and is configured, after triggering, to provide an ejection force to the push rod 8, and, after the push rod 8 disengages from the spring clip 11, to provide an ejection force to the spring clip 11.

In the present embodiment, the spring clip 11 is mounted between the support frame 7 and the push rod 8 and is configured, after completion of injection, to be ejected under driving of the activation spring 9 to strike the rear cover 12 to produce a sound. Specifically, the spring clip 11 has a longitudinal cross-section of an E-shaped structure; a front end of the spring clip 11 is provided with an engagement portion 111 for cooperatively positioning with the support frame 7; the engagement portion 111 is initially press-fitted by the push rod 8 and engaged with the support frame 7; a positioning rod 112 is provided at a middle of the spring clip 11; and the positioning rod 112 is threaded through the push rod 8 and is configured to position and mount the activation spring 9.

In the present embodiment, the rear cover 12 is fixed to the rear end of the support frame 7 and is configured to be struck by the spring clip 11 to produce a sound. Specifically, the rear cover 12 is symmetrically provided with connecting catches 121 for engaging with the second positioning groove 76 on the support frame 7.

In the present embodiment, an inner wall of the first positioning groove 71, an inner wall of the second positioning groove 76, an inner wall of the third positioning groove 81, and the engagement portion 111 are each provided with an inclined surface structure for disengagement from the initial position.

During assembly of the activation assembly of the present invention, the protective sleeve 10 is mounted outside the support frame 7, at which time the second positioning catch 103 is engaged within the first positioning groove 71, the protrusion 77 is located within the first sliding slot 104, and the first cantilever 74 is press-fitted by the protective sleeve 10; the spring clip 11 is mounted into the support frame 7, at which time the engagement portion 111 is engaged with and positioned by the positioning portion 73; the activation spring 9 is sleeved on the positioning rod 112, and then the push rod 8 is mounted into the support frame 7, at which time the first positioning catch 75 is engaged within the third positioning groove 81; the rear cover 12 is engaged with the rear end of the support frame 7, and the connecting catch 121 on the rear cover 12 is engaged within the second positioning groove 76.

During assembly of the front portion of the pre-filled auto-injector pen, the return spring 4 is mounted into the activation sleeve 3; then the activation sleeve 3 is mounted into the housing 2; the pen cap 1 is then mounted into the housing 2 to cover the activation sleeve 3; the fixing seat 6 is then mounted into the housing 2 to cooperatively position with the activation sleeve 3, at which time the second cantilever 61 of the fixing seat 6 is located within the slot hole 33 of the activation sleeve 3, and the outer catch 62 is initially cooperatively positioned with the protection catch 34; the pre-filled syringe 5 is mounted into the fixing seat 6, at which time the inner catch 63 cooperates with the protruding tail end 52 for limiting. Finally, the assembled activation assembly is mounted into the housing 2 and is fixedly mounted by engagement of the support frame 7 with the housing 2.

During use of the present invention, the pen cap 1 is pulled off to detach the inner protective rubber sleeve 51 from the pre-filled syringe 5. The activation sleeve 3 is pressed down, the activation sleeve 3 moves rearward, the activation portion 32 of the activation sleeve 3 contacts the linkage portion 102 of the protective sleeve 10, the protective sleeve 10 moves rearward under driving of the activation sleeve, the second positioning catch 103 disengages from the first positioning groove 71, the protective sleeve 10 continues to move rearward, and the second positioning catch 103 reaches the limiting portion 72; at this time, the first sliding slot 104 moves to the position of the first cantilever 74, the first cantilever 74 loses its press-fitting force, and under action of the activation spring 9, the push rod 8 spreads the first positioning catch 75 outward to disengage from the third positioning groove 81, and the push rod 8 moves forward along the inner wall of the support frame 7; meanwhile, the protection catch 34 of the activation sleeve 3 is displaced upward, the second cantilever 61 on the fixing seat 6 gains a degree of freedom toward the housing 2, the slot hole 33 on the activation sleeve 3 provides space for deformation of the second cantilever 61, the push rod 8 is displaced downward under the elastic force of the activation spring 9, pushing the pre-filled syringe 5 to break through the inner catch 63 and to move downward, and the needle is exposed beyond the activation sleeve 3 and enters the skin; the push rod 8 continues to move downward to push the rubber plunger inside the pre-filled syringe 5 to perform injection; when the push rod 8 moves to a limit position, i.e., when injection is completed, the push rod 8 completely disengages from the spring clip 11; at this time, under action of the activation spring 9, the engagement portion 111 of the spring clip 11 disengages from the positioning portion 73 of the support frame 7, and the spring clip 11 is ejected and strikes the rear cover 12 to produce a sound, indicating that injection has been completed.

Meanwhile, the deformable front end 101 of the protective sleeve 10 is spread and deformed when moving to the protrusion 77, thereby restricting the rearward movement path of the activation sleeve 3; that is, the activation sleeve 3, after needle withdrawal is completed, is in a state of protecting the needle, and cannot be operated for a second time or cause accidental injury.

In the present invention, activation of the push rod 8 can be achieved merely through movement of the protective sleeve 10, and the activation is reliable and stable. After completion of push-injection by the push rod 8, the push rod 8 disengages from the spring clip 11, and the spring clip 11 is capable, under action of the activation spring 9, of moving rearward to strike the rear cover 12 to produce a sound, thereby achieving the injection-completion reminder function and ensuring reliability of the end prompt. The protective sleeve 10 is capable of restricting rearward movement of the activation sleeve 3 after injection completion, thereby achieving needle protection, preventing accidental injury, and disabling secondary use. The present invention has a viewing window function, and the injection condition can be observed after the end prompt sound, thereby ensuring continuous integrity of the injection.

## Claims

1. An activation assembly for a pre-filled auto-injector pen, comprising:
a support frame (7) fixed within a housing (2) and configured to initially define initial positions of a push rod (8), a spring clip (11), and a protective sleeve (10);
the push rod (8), mounted within the support frame (7) and initially positioned by the support frame (7), and configured, after the protective sleeve (10) is triggered, to be driven under driving of an activation spring (9) to drive a pre-filled syringe (5) to move so as to complete automatic needle insertion;
the protective sleeve (10), mounted outside the support frame (7), initially engaged outside the support frame (7) and capable, after being moved under driving of an activation sleeve (3), of triggering the push rod (8), and further capable, after movement, of restricting rearward movement of the activation sleeve (3) for needle protection;
the activation spring (9), mounted inside the push rod (8) via the spring clip (11) and configured, after triggering, to provide an ejection force to the push rod (8), and, after the push rod (8) disengages from the spring clip (11), to provide an ejection force to the spring clip (11);
the spring clip (11), mounted between the support frame (7) and the push rod (8) and configured, after completion of injection, to be ejected under driving of the activation spring (9) to strike a rear cover (12) to produce a sound; and
the rear cover (12), fixed to a rear end of the support frame (7) and configured to be struck by the spring clip (11) to produce a sound.

2. The activation assembly for a pre-filled auto-injector pen according to claim 1, wherein the support frame (7) is provided with a first positioning groove (71) for cooperatively positioning with the protective sleeve (10) initially; the support frame (7) is further provided, at a rear side of the first positioning groove (71), with a limiting portion (72) for cooperatively positioning with the protective sleeve (10) after movement; a front end of the support frame (7) is provided with a positioning portion (73) for cooperatively positioning with the spring clip (11) initially; the support frame (7) is provided with a first cantilever (74); an inner wall of the first cantilever (74) is provided with a first positioning catch (75) for cooperatively positioning with the push rod (8) initially; the first cantilever (74) is initially press-fitted by the protective sleeve (10) such that the first positioning catch (75) engages with the push rod (8); a rear end of the support frame (7) is provided with a second positioning groove (76) for fixing the rear cover (12); and an outer surface of the support frame (7) is symmetrically provided with arc-shaped protrusions (77) for spreading open the protective sleeve (10) to abut against the activation sleeve (3).

3. The activation assembly for a pre-filled auto-injector pen according to claim 2, wherein the protective sleeve (10) has a deformable front end (101); the deformable front end (101) is capable of being spread open under action of the support frame (7) to abut against a rear end of the activation sleeve (3); a linkage portion (102) is protrudingly provided on an outer side surface of the deformable front end (101); the linkage portion (102) is driven by the rear end of the activation sleeve (3) to drive the protective sleeve (10) to move rearward; a second positioning catch (103) is provided on an inner side of a rear end of the protective sleeve (10); the second positioning catch (103) is capable of engaging with the support frame (7) both initially and after movement; and the protective sleeve (10) is provided with a first sliding slot (104) for initially receiving the protrusions (77) and providing spreading space for the first cantilever (74) after movement.

4. The activation assembly for a pre-filled auto-injector pen according to claim 2, wherein the push rod (8) is provided with a third positioning groove (81) for cooperative initial positioning with the first positioning catch (75).

5. The activation assembly for a pre-filled auto-injector pen according to claim 1, wherein the spring clip (11) has a longitudinal cross-section of an E-shaped structure; a front end of the spring clip (11) is provided with an engagement portion (111) for cooperatively positioning with the support frame (7); the engagement portion (111) is initially press-fitted by the push rod (8) and engaged with the support frame (7); a positioning rod (112) is provided at a middle of the spring clip (11); and the positioning rod (112) is threaded through the push rod (8) and is configured to position and mount the activation spring (9).

6. A pre-filled auto-injector pen, comprising:
a housing (2) having a hollow structure;
a pen cap (1) engaged with a head of the housing (2) and capable, when pulled off, of driving an inner protective rubber sleeve (51) of a pre-filled syringe (5) to detach therewith;
an activation sleeve (3) movably mounted within a housing (2), with a return spring (4) for resetting being axially provided between the activation sleeve (3) and the housing (2); a head of the activation sleeve (3) being exposed outside the housing (2), and the activation sleeve (3) being capable, when moving from an initial position to a limit position, of triggering an activation assembly;
the pre-filled syringe (5), clamped on a fixing seat (6) when pre-mounted, wherein during movement of the activation sleeve (3) from the initial position to the limit position, an injection end of the pre-filled syringe (5) is always concealed within a needle shield, and when the injection end of the pre-filled syringe (5) is triggered, the pre-filled syringe (5) is instantaneously displaced to extend beyond the activation sleeve (3) to achieve automatic needle insertion;
the fixing seat (6), fixedly mounted between the activation sleeve (3) and the pre-filled syringe (5), configured to initially define an initial position of the pre-filled syringe (5) and to provide support during automatic needle insertion of the pre-filled syringe (5);
wherein the activation assembly for a pre-filled auto-injector pen according to any one of claims 1-5 is mounted within the housing (2).

7. The pre-filled auto-injector pen according to claim 6, wherein a head of the push rod (8) is a pushing end (82) in contact with a rubber plunger of the pre-filled syringe (5), and an interior of the push rod (8) is a hollow structure for accommodating the activation spring (9).

8. The pre-filled auto-injector pen according to claim 6, wherein the activation sleeve (3) comprises an activation sleeve body (31) for protecting the needle; a tail end of the activation sleeve body (31) is provided with symmetrically arranged activation portions (32); each of the activation portions (32) is provided with a slot hole (33) for movement of a second cantilever (61) of the fixing seat (6); and an inner side of each of the activation portions (32) is provided, at one end of the slot hole (33), with a protection catch (34) protruding radially for defining an initial position of the second cantilever (61) of the fixing seat (6).

9. The pre-filled auto-injector pen according to claim 8, wherein the fixing seat (6) is symmetrically provided with second cantilevers (61); the second cantilevers (61) are arranged corresponding to the slot holes (33) of the activation sleeve (3); an outer side of an end portion of each of the second cantilevers (61) is provided with an outer catch (62) for initially cooperating with the protection catch (34) of the activation sleeve (3) to define a position of the second cantilever (61); and an inner side of the end portion of each of the second cantilevers (61) is provided with an inner catch (63) for initially cooperating with a protruding tail end (52) of the pre-filled syringe (5) to support the pre-filled syringe (5); an inner wall of the fixing seat (6) is provided with a stepped surface (64) for limiting movement of the pre-filled syringe (5); and the inner wall of the fixing seat (6) is axially provided, from the stepped surface (64) to a tail end thereof, with vertical ribs (65) for providing support during automatic needle insertion of the pre-filled syringe (5).

10. The pre-filled auto-injector pen according to claim 6, wherein the housing (2) is provided with a viewing window (21) for inspecting a state of medicinal liquid, whether a position of the pre-filled syringe (5) is correct, and an infusion condition after completion of injection.

11. The pre-filled auto-injector pen according to claim 6, wherein the pen cap (1) is provided with symmetrically arranged pen cap catches (1a), and the pen cap catches (1a) are initially snap-connected with the inner protective rubber sleeve (51) of the pre-filled syringe (5).

12. The pre-filled auto-injector pen according to claim 6, wherein the rear cover (12) is symmetrically provided with connecting catches (121) for engaging with the second positioning groove (76) on the support frame (7).
